(19)

Europäisches
Patentamt
European
Patent Office
Office européen
des brevets

(11) **EP 4 039 243 A1**

(12) **EUROPEAN PATENT APPLICATION**

(43) Date of publication:
 **10.08.2022 Bulletin 2022/32**

(21) Application number: **22155125.2**

(22) Date of filing: **04.02.2022**

(51) International Patent Classification (IPC):
 ***A61K 8/04*** *(2006.01)*    ***A61K 8/73*** *(2006.01)*
 ***A61K 8/34*** *(2006.01)*    ***A61K 8/36*** *(2006.01)*
 ***A61Q 5/00*** *(2006.01)*

(52) Cooperative Patent Classification (CPC):
 **A61K 8/046; A61K 8/345; A61K 8/361;**
 **A61K 8/731; A61Q 5/00;** A61K 2800/10

(84) Designated Contracting States:
 **AL AT BE BG CH CY CZ DE DK EE ES FI FR GB
 GR HR HU IE IS IT LI LT LU LV MC MK MT NL NO
 PL PT RO RS SE SI SK SM TR**
 Designated Extension States:
 **BA ME**
 Designated Validation States:
 **KH MA MD TN**

(30) Priority: **05.02.2021 JP 2021017254**

(71) Applicant: **SHIN-ETSU CHEMICAL CO., LTD.
 Tokyo 1000005 (JP)**

(72) Inventors:
 • **SUDO, Takane
  Joetsu-shi, 9428601 (JP)**
 • **NARITA, Mitsuo
  Niigata, 9428601 (JP)**

(74) Representative: **Schulz Junghans
 Patentanwälte PartGmbB
 Großbeerenstraße 71
 10963 Berlin (DE)**

(54) **COMPOSITION FOR AEROSOL COSMETICS, AND AEROSOL COSMETICS**

(57)    The objective of the present invention is to provide a composition for aerosol cosmetics employed for producing aerosol cosmetics which easily render the formulation design, have improved dispensability, and stably make a foam with greater foam quality. The objective is achieved by a composition for aerosol cosmetics, comprising hydroxypropyl methylcellulose, an organic solvent and water, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution, and the like.

EP 4 039 243 A1

**Description**

Technical Field

The present invention relates to a composition for aerosol cosmetics and aerosol cosmetics.

Background Art

Hair cosmetics and other cosmetics have been known as aerosol type cosmetics. Aerosol cosmetics can easily make a foam just by being dispensed from the container.

It is preferred that the foam of aerosol cosmetics made after being dispensed is elastic and has a bouncy foam feeling, and that aerosol cosmetics have an improved foam stability.

On the other hand, for the purpose of improvement of the foam, such as ease of foaming, foam quality and foam stability, aerosol skin cleansers containing a combination of various surfactants and cellulose type thickening compounds have been used.

For example, it has been reported that a foam type skin cleanser contains an undiluted liquid and a propellant filled in an aerosol container, which is designed to make a fine, firm and dense foam while suppressing the skin tightness after washing, wherein the undiluted liquid contains a higher fatty acid and/or salt thereof, a fatty acid ester oil in the solid form at room temperature, water, and a sorbitan fatty acid ester type nonionic surfactant (for example, see Patent Document 1).

Patent Document 1 reports that the above-mentioned undiluted liquid further contains a thickening compound selected from xanthan gum and guar gum as well as a thickening compound selected from hydroxypropyl methylcellulose, hydroxyethyl cellulose and hydroxypropyl cellulose so that the viscosity of the undiluted liquid can increase, and the resulting foam type skin cleanser can have improved foam stability.

Citation List

Patent Documents

Patent Document 1: JP 2019-59720 A

Summary of the Invention

Problems to be solved by the Invention

Aerosol cosmetics are desired to form a foam which is not defoamed while dispensed at high pressure. The viscoelasticity of a foam film is involved in the formation of such foam. However, when various thickening agents are combined as in Patent Document 1, the content of each thickening agent and the ratio between the thickening agents affect the viscoelasticity of the foam film. Therefore, many combinations of various thickening agents should be subjected to extensive trial and error in order to find out the adequate content and ratio, which leads to a problem of complicating the formulation design.

In addition, the combination of thickening agents without the adequate content and ratio may cause problems such as deterioration of the dispensability due to increased viscosity; poor foaming due to inhibition of surfactant adsorption; and degradation of foam stability.

Furthermore, even if hydroxypropyl methylcellulose, which is described in Patent Document 1, is employed as a thickening agent, the foam elasticity and stability may be deteriorated.

In view of the above circumstances, it is an object of the present invention to provide aerosol cosmetics which easily render the formulation design, have improved dispensability, and stably make a foam with greater foam quality, and a composition for aerosol cosmetics employed for producing such aerosol cosmetics.

Means of solving the Problems

In order to solve the above identified problems, the present inventors have deeply studied various factors that affect the viscoelasticity of foam film. In the course of extensive efforts through trial and error for such various factors, the present inventors produced aerosol cosmetics using hydroxypropyl methylcellulose (hereinafter also referred to as "HPMC"), which has a predetermined ratio of interfacial elastic modulus to interfacial viscous modulus, and surprisingly found that such aerosol cosmetics have improved dispensability and can make a foam with greater foam quality by having improved foam elasticity and stability as well as a bouncy foam feeling.

More surprisingly, the present inventors found that the properties of such aerosol cosmetics can be achieved even if the content and ratio of surfactant and organic solvent employed were changed, resulting in simplifying the formulation design.

Based on the above findings, the present inventors have successfully invented a composition for aerosol cosmetics that contains hydroxypropyl methylcellulose having a predetermined ratio of interfacial elastic modulus to interfacial viscous modulus, and aerosol cosmetics containing such composition for aerosol cosmetics. As such, the present invention has been completed on the basis of the findings and successful examples that were first found or obtained by the present inventors.

According to the present invention, a composition for aerosol cosmetics, aerosol cosmetics, and methods of producing them are provided in the following aspects:

[1] a composition for aerosol cosmetics, containing hydroxypropyl methylcellulose, an organic solvent and water, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution;

[2] the composition for aerosol cosmetics according to [1] above, wherein the interfacial viscous modulus (E") of the hydroxypropyl methylcellulose is equal to or higher than 15.0 mN/m when measured with a tensiometer at 25°C using a 0.2% by mass aqueous solution;

[3] the composition for aerosol cosmetics according to [1] or [2] above, wherein the interfacial elastic modulus (E') of the hydroxypropyl methylcellulose is in a range between 80.0 mN/m and 250.0 mN/m when measured with a tensiometer at 25°C using a 0.2% by mass aqueous solution;

[4] the composition for aerosol cosmetics according to any one of [1] to [3] above, wherein the viscosity of the hydroxypropyl methylcellulose is in a range between 1,000 mPa.s and 100,000 mPa.s when measured with a viscometer at 20°C using a 2% by mass aqueous solution;

[5] the composition for aerosol cosmetics according to any one of [1] to [4] above, further containing a surfactant;

[6] aerosol cosmetics containing the composition for aerosol cosmetics according to any one of [1] to [5] above and a propellant in an aerosol container;

[7] a method of producing a composition for aerosol cosmetics, including the step of mixing a solution containing an organic solvent and water with hydroxypropyl methylcellulose to obtain the composition for aerosol cosmetics, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution; and

[8] a method of producing aerosol cosmetics, including the steps of mixing a solution comprising an organic solvent and water with hydroxypropyl methylcellulose to obtain the composition for aerosol cosmetics; and filling the composition for aerosol cosmetics and a propellant in an aerosol container to obtain the aerosol cosmetics, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution.

Effects of the Invention

According to the present invention, it is possible to produce aerosol cosmetics that have improved dispensability, and stably make a foam with greater foam quality, such as excellent foam elasticity and stability (foam holding), as well as the desirable bouncy foam feeling, without a complicated formulation design in terms of content or ratio of a surfactant and an organic solvent employed.

Description of Embodiments

While each embodiment of the present invention will now be described in detail, the present invention may take various forms to the extent that its objective can be achieved.

Unless otherwise specified, each term used herein is used in the meaning commonly used by those skilled in the art, in particular in the cellulose and cosmetics fields, and should not be construed to have any meaning that is unduly limiting. Also, any speculations and theories herein are made on the basis of the knowledge and experiences of the present inventors and as such, the present invention is not bound by any such speculations and theories.

While the term "composition" is not particularly limited and means composition as well known, it is, for example, comprised of a combination of two or more components. The term "and/or" as used herein means either any one of, any combination of two or more of, or combination of all of the listed related items.

The term "content" is synonymous with concentration and amount used (amount added), and means the ratio of the amount of a component relative to the total amount of the composition. Unless otherwise specified, the unit of content used herein means "% by mass" and "%(w/w)". However, the total content of the components may not exceed 100%.

The wording "to" for indicating a range of values is intended to include values preceding and following the wording; for example, "0% to 100%" means a range from 0% or more and 100% or less. The terms "more than" and "less than" used herein means the lower and upper limits without including a value following the term, respectively. For example, "more than 1" means a value beyond 1, and "less than 100" means a value below 100.

The terms "include," "comprise," and "contain" mean that an element(s) other than an element(s) as explicitly indicated can be added as inclusions, which are, for example, synonymous with "at least include," but encompasses the meaning of "consist of" and "substantially consist of". In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

The number of digits of an integer equals to its significant figure. For example, 1 has one significant figure and 10 has two significant figures. For a decimal number, the number of digits after a decimal point equals to its significant figure. For example, 0.1 has one significant figure and 0.10 has two significant figures.

Aerosol cosmetics are cosmetics which contain cosmetic components and a propellant (pressurized gas) so as to coexist in a pressure-resistant container. The gas in the cosmetic components expands through the release of pressure so that the aerosol cosmetics can take on its foaming form.

Composition for aerosol cosmetics

The composition for aerosol cosmetics in one embodiment of the present invention contains hydroxypropyl methylcellulose, an organic solvent and water, wherein the ratio (E'/E") of interfacial elastic modulus (E') to interfacial viscous modulus (E") of hydroxypropyl methylcellulose is a predetermined value.

(1) Hydroxypropyl methylcellulose (HPMC)

After repeated trials and failures, the present inventors have directed their attention to the interfacial viscous modulus (E") and the interfacial elastic modulus (E') of an aqueous solution of HPMC. The interfacial viscous modulus (E") corresponds to an index of viscosity term in viscoelasticity of an aqueous solution of HPMC. The interfacial elastic modulus (E') corresponds to an index of elasticity term in viscoelasticity of an aqueous solution of HPMC. It is believed that with respect to a continuous foam generated continuously from a composition containing HPMC, the interfacial elastic modulus (E') affects the elasticity (hardness) of the foam surface while the interfacial viscous modulus (E") affects the viscosity (buffering force) of the foam surface.

The viscoelasticity of a phase boundary between an aqueous solution and a gas can be regarded as the same as the viscoelasticity of a solid. The interfacial viscoelasticity modulus E*, the interfacial elasticity modulus E', and the interfacial viscosity modulus E" are given by the following equations (1) and (2).

Equation 1

$$E^* = E' + iE'' \quad \cdots (1)$$

$$|E^*| = \sqrt{E'^2 + E''^2} = E \quad \cdots (2)$$

In the above equations (1) and (2), the interfacial elasticity modulus E' means the change in surface tension relative to the change in area, and the interfacial viscosity modulus E" means the change in surface tension relative to the velocity of change in area. In the present description, E' is also referred to as interfacial elasticity modulus and E" as interfacial viscosity modulus.

In the case of foam, the interfacial elasticity modulus (E') is the back-driving force generated when the foam film surface elongates. A high E' can achieve a hard foam film, making foam coalescence and defoaming less likely, and improve foamability and foam stability.

The interfacial viscosity modulus (E") correlates with the diffusion rate of molecules in the foam film. A high E" quickly lowers a surface tension of foam, which improves foam stability by maintaining high foaming speed and surface tension uniformity.

In general, if E' is high, the concentration gradient of the surfactant can be cancelled out, which can keep the surface tension constant, resulting in improved foam stability. The present inventors assumed that the balance between E' and E" in an aqueous solution of a component employed as a foaming agent would affect the hardness of the foam film after foaming, since the foam is required to be made but not defoamed while dispensing a composition for aerosol cosmetics at high pressure in the course of foaming of an aerosol. The present inventors used HPMC with E' and E" in a predetermined balance, and finally found that aerosol cosmetics can be obtained as one improving dispensability and stably making a foam with an excellent foam quality.

Taking into consideration the above circumstances, in HPMC employed as a foaming agent, the ratio (E'/E") of the interfacial elastic modulus (E') to the interfacial viscous modulus (E") is preferably a value equal to or more than 5.0, more preferably from 5.0 to 17.0, still more preferably from 5.5 to 9.0, and still even more preferably from 5.7 to 9.0 or 5.7 to 8.6 in terms of foamability and stability of the foam film generated. When the composition for aerosol cosmetics obtained by using HPMC having the ratio E'/E" of less than 5.0 is foamed, the resulting foam tends to have poor foam hardness, lower foam height, and/or a less bouncy foam feeling. Furthermore, the composition for aerosol cosmetics obtained by using HPMC having a ratio E'/E" of less than 5.0 tends to have inferior dispensability compared to the composition for aerosol cosmetics obtained by using HPMC having the ratio E'/E" of 5.0 or more.

Preparation of a 0.2% by mass aqueous HPMC solution, measurements of the interfacial viscous modulus (E") and the interfacial elastic modulus (E'), and calculation of their ratio (E'/E") are performed as described in Examples below.

As long as the ratio (E'/E") of the interfacial elastic modulus (E') to the interfacial viscous modulus (E") of 0.2% by mass aqueous HPMC solution at 25°C is a value equal to or more than 5.0, the value of each of the interfacial elastic modulus (E') and the interfacial viscous modulus (E") is not particularly limited. Taking into consideration foamability and foam stability after foaming, the interfacial elastic modulus (E') and the interfacial viscous modulus (E") of 0.2% by mass aqueous HPMC solution at 25°C preferably have a value within the predetermined range, respectively.

For example, the interfacial viscous modulus (E") of 0.2% by mass aqueous HPMC solution at 25°C is preferably a value equal to or more than ($\geq$) 15.0 mN/m, more preferably from 15.0 mN/m to 40.0 mN/m, still more preferably from 15.0 mN/m to 35.0 mN/m, and still even more preferably from 15.0 mN/m to 28.0 mN/m.

In certain embodiments, the interfacial viscous modulus (E") of HPMC employed according to the invention, measured in 0.2% (w/w) in aqueous solution at 25°C, is $\geq$ 15.5 mN/m. In certain preferred embodiments, the interfacial viscous modulus (E") of HPMC employed according to the invention, measured in 0.2% (m/m) in aqueous solution at 25°C, is $\geq$ 15.7 mN/m.

In certain embodiments, the value of E" of the HPMC employed according to the invention, measured in 0.2% (w/w) in aqueous solution at 25°C, is $\leq$ 40.0 mN/m. In certain preferred embodiments, this E" is $\leq$ 35.0 mN/m. In certain more preferred embodiments, this E" is $\leq$ 28.0 mN/m. Any of the upper and lower limits for E" given can be combined to ranges in which the invention can be favourably practiced.

In certain embodiments, this value of E" ranges from 15.3 mN/m to 35.0 mN/m. In certain embodiments, this value of E" ranges from 15.5. mN/m to 28.0 mN/m.

HPMC with E" of less than 15.0 mN/m may not provide sufficient foamability and foam stability after foaming to the composition for aerosol cosmetics, which in turn makes it difficult to generate a continuous foam and tends to break the resulting foam easily. As a result, the obtained aerosol cosmetics may not foam sufficiently and the resulting foam may dissipate prematurely, and a foam with bouncy foam feeling may not be obtained.

The interfacial elastic modulus (E') of 0.2% by mass aqueous HPMC solution at 25°C is preferably from 80.0 mN/m to 250.0 mN/m, more preferably from 85.0 mN/m to 230.0 mN/m, still more preferably from 90.0 mN/m to 210.0 mN/m, and still even more preferably from 100.0 mN/m to 208.0 mN/m or 100.0 mN/m to 208.0 mN/m. HPMC with E' of less than 80.0 mN/m may not provide foam stability after foaming to the composition for aerosol cosmetics.

The combination of the ratio (E'/E") of the interfacial elastic modulus (E') to the interfacial viscous modulus (E"), the interfacial elastic modulus (E') and the interfacial viscous modulus (E") of 0.2% by mass aqueous HPMC solution at 25°C is preferably the combination of E'/E" of 5.3 to 16.7, E" equal to or more than 15.0 mN/m, and E' of 80.0 mN/m to 250.0 mN/m; more preferably the combination of E'/E" of 5.3 to 17.0, E" of 15.0 mN/m to 40.0 mN/m, and E' is 85.0 mN/m to 230.0 mN/m; still more preferably the combination of E'/E" of 5.5 to 9.0, E" of 15.0 mN/m to 35.0 mN/m, and E' of 90.0 mN/m to 210.0 mN/m; and still even more preferably the combination of E'/E" of 5.7 to 9.0 or 5.7 to 8.6, E" of 15.0 mN/m to 28.0 mN/m or 15.5 mN/m to 28.0 mN/m, and E' of 100.0 mN/m to 210.0 mN/m or 100.0 mN/m to 208.0 mN/m, from the viewpoint of foamability and foam stability after foaming.

The aqueous solution containing HPMC with E'/E" of 5.0 or more tends to have a higher viscosity than that of the aqueous solution containing HPMC with E'/E" of less than 5.0. Therefore, the viscosity of 2.0% by mass aqueous HPMC solution at 20°C is preferably from 1,000 mPa·s to 100,000 mPa·s, more preferably from 1,000 mPa·s to 75,000 mPa·s, still more preferably from 2,000 mPa·s to 50,000 mPa·s, and still even more preferably from 3,500 mPa·s to 20,000 mPa·s in terms of control of the viscosity of the composition for aerosol cosmetics.

Since the viscosity of 2.0% by mass aqueous HPMC solution at 20 °C is a value equal to or more than 600 mPa.s, the viscosity is measured using a single cylinder-type rotational viscometer according to "2. Method II Viscosity measurement by rotational viscometer" in the Viscosity Determination in General Tests described in Japanese Pharmacopoeia, 17th edition, as described in Examples below.

It is preferable that HPMC has advantageous properties as a component of the composition for aerosol cosmetics.

For example, the degree of substitution (DS) of methoxy groups in HPMC is preferably in the range between 1.00 and 2.20, more preferably between 1.50 and 2.00, and still more preferably between 1.60 and 1.95, from the viewpoint of surfactant action.

DS of methoxy groups in HPMC refers to the average number of methoxy groups per unit of anhydrous glucose.

The molar substitution (MS) of hydroxypropoxy groups in HPMC is preferably in the range between 0.10 and 0.60, and more preferably between 0.15 and 0.35, from the viewpoint of surfactant action.

MS of hydroxypropoxy groups in HPMC refers to the average number of moles of hydroxypropoxy groups per mole of anhydrous glucose.

The combination of DS of methoxy groups and MS of hydroxypropoxy groups in HPMC is preferably DS of 1.2 to 2.1 and MS of 0.1 to 0.6; more preferably DS of 1.5 to 2.1 and MS of 0.1 to 0.4; and still more preferably DS of 1.5 to 2.0 and MS of 0.1 to 0.4, from the viewpoint of surfactant action.

DS of methoxy groups and MS of hydroxypropoxy groups in HPMC are determined by converting the values measured by the measurement method for Hypromellose (Hydroxypropyl Methylcellulose) described in Japanese Pharmacopoeia, 17th edition.

As long as HPMC in which the ratio (E'/E") of the interfacial elastic modulus (E') to the interfacial viscous modulus (E") is equal to or higher than 5.0 when measured with a tensiometer at 25°C using a 0.2% by mass aqueous solution can be obtained, HPMC may be obtained by any method that is not particularly limited. Such HPMC can be produced by, for example, an HPMC production method including the steps of continuously and simultaneously feeding and mixing a powdered pulp and an alkali metal hydroxide aqueous solution into a high-speed dispersing device operated at a peripheral speed of 5 m/s or higher on the agitator blade to obtain an alkali cellulose; subjecting the alkali cellulose, a methylating agent and a hydroxypropylating agent to etherification reaction, resulting in a crude product of hydroxypropyl methylcellulose; and washing and drying the crude product of hydroxypropyl methylcellulose to obtain hydroxypropyl methylcellulose (HPMC). The above method will now be described in further detail.

First, an alkali cellulose is obtained by continuously and simultaneously feeding and mixing a powdered pulp and an alkali metal hydroxide aqueous solution into a high-speed dispersing device operated at a peripheral speed of 5 m/s or higher on the agitator blade.

The powdered pulp is continuously fed into the high-speed dispersing device, mixed with an alkali metal hydroxide aqueous solution, which is simultaneously and continuously fed from the same or different feed opening, preferably from a different feed opening, and then the resulting alkali cellulose is continuously discharged from the discharge opening of the high-speed dispersing device.

Pulp is not particularly limited to, but includes, for example, wood pulp, linter pulp, and other cellulose pulps. Pulp is preferably a powdered pulp obtained by pulverizing treatment or other treatments.

The properties of powdered pulp are not particularly limited, but for example, the weight average particle size (D50) of powdered pulp is preferably in the range between 30 μm and 400 μm, and more preferably between 30 μm and 200 μm, from the viewpoint of mixability in the reaction container. The moisture content in powdered pulp is preferably in the range between 0% by mass and 15% by mass, and more preferably between 0% by mass and 6% by mass, from the viewpoint of suppressing side reactions among water, methylating agent and hydroxypropylating agent thereby preventing the utilization rate of them from reducing. The viscosity of powdered pulp is preferably in the range between 100 ml/g and 10,000 ml/g, and more preferably between 500 ml/g and 3,000 ml/g, from the viewpoint of tending to affect the interfacial viscous modulus (E"), the interfacial elastic modulus (E') and the ratio (E'/E") thereof of the resulting HPMC. The values of D50, moisture content, and viscosity of powdered pulp are measured by the methods as described in Examples below.

Examples of alkali metal hydroxide aqueous solution include sodium hydroxide aqueous solution and potassium hydroxide aqueous solution. Sodium hydroxide aqueous solution is preferred from an economic viewpoint.

The concentration of alkali metal in alkali metal hydroxide aqueous solution is preferably in the range between 10% by mass and 60% by mass, and more preferably between 35% by mass and 55% by mass in terms of economics and ease of handling.

The temperature of alkali metal hydroxide aqueous solution is preferably in the range between 10°C and 80°C, and more preferably between 15°C and 60°C, from the viewpoint of preventing the polymerization degree of pulp from decreasing.

The amount of alkali metal hydroxide aqueous solution used (mass of alkali metal hydroxide aqueous solution used relative to unit mass of powdered pulp used) can be set appropriately depending on the DS of the methoxy groups and the MS of the hydroxypropoxy groups in HPMC to be produced. For example, if it is desired to obtain HPMC with a higher DS of the methoxy groups, the amount used may be larger, and if it is desired to obtain HPMC with a lower DS of the methoxy groups, the amount used may be smaller. Specifically, for a unit part by mass (1.00 part by mass) of powdered pulp, the amount used is preferably in the range between 0.20 parts by mass and 4.00 parts by mass, more preferably between 0.30 parts by mass and 3.00 parts by mass, and still more preferably between 0.30 parts by mass and 1.50 parts by mass.

The high-speed dispersing device may be equipped with a feed opening for powdered pulp and a feed opening for alkali metal hydroxide solution, a discharge opening for alkali cellulose, an agitator blade, and a mixing container, so that the fed powdered pulp and alkali metal hydroxide solution can be dispersed uniformly.

The type of agitator blade is not particularly limited to, but includes, for example, a cylindrical type with pin, a cone type with pin, a disk type with pin, a cylindrical type with scraping blade, and a disk type with scraping blade.

As long as the fed powdered pulp and alkali metal hydroxide aqueous solution are appropriately contacted and mixed, the peripheral speed on agitator blade is not particularly limited. For example, the peripheral speed is preferably equal to or more than 5 m/s, and more preferably in the range between 7 m/s and 50 m/s. If the peripheral speed on agitator blade is less than 5 m/s, the distribution of alkali in alkali cellulose may become unhomogenous, and as a result, the distribution of substituents and/or the distribution of molecular weight after etherification may become unhomogenous. Accordingly, HPMC of which the interfacial viscous modulus, the interfacial elastic modulus and the ratio thereof fall into the above-mentioned range may not be obtained.

The peripheral speed on agitator blade is the speed of the fastest part on an agitator blade rotating in a high-speed dispersing device (i.e., the speed of the outermost circumference of agitator blade).

The peripheral speed v (m/s) on agitator blade is calculated from the diameter d (mm) of the agitator blade and the rotational speed n (rpm (revolutions per minute)) of the agitator blade, using the following formula.

$$v = \pi \times d \times n / 60,000$$

Examples of a high-speed dispersing device include, but are not limited to, a flow jet mixer (e.g., manufactured by Funken Powtex) and a micro speed mixer (e.g., manufactured by Takara Koki).

The powdered pulp and the alkali metal hydroxide aqueous solution are continuously and simultaneously fed into the high-speed dispersing device.

It is preferable to use a quantitative feeder for feeding the powdered pulp to the high-speed dispersing device from the viewpoint of maintaining quantitativeness during feeding. Examples of quantitative feeder include, but are not limited to, an auto feeder (e.g., manufactured by Funken Powtex), a circle feeder (e.g., manufactured by Yoshikawa), and a vibratory feeder (e.g., manufactured by Symphonia Technology).

It is also preferable to use a quantitative pump for feeding the alkali metal hydroxide aqueous solution to the high-speed dispersing device from the viewpoint of maintaining quantitativeness during feeding. Examples of quantitative pump include, but are not limited to, a Mohnos pump (e.g., manufactured by Heishin) and a diaphragm pump (e.g., manufactured by Teikoku Electric MFG).

The alkali metal hydroxide aqueous solution may be fed from either the same or different feed opening as the feed opening of powdered pulp. From the viewpoint of preventing the feed opening from being blocked due to clumping, it is preferable to feed the alkali metal hydroxide aqueous solution from a feed opening in a different location than the feed opening of powdered pulp.

The retention time of the alkali metal hydroxide aqueous solution or the powdered pulp in the mixing container of the high-speed dispersing device is not particularly limited. For example, the retention time is preferably in the range between 0.1 seconds and 20 seconds, from the viewpoint of productivity and uniformity of alkali distribution in the resulting alkali cellulose.

Optionally, a lower primary alcohol such as methanol and ethanol, and an inert solvent such as dimethyl ether may be supplied in addition to the alkali metal hydroxide aqueous solution and powdered pulp from the viewpoint of improving uniformity of alkali metal hydroxide in the resulting alkali cellulose.

Furthermore, if desired, the polymerization degree of alkali cellulose may be controlled by placing the high-speed dispersing device under a vacuum and/or nitrogen atmosphere to prevent the resulting alkali cellulose from decreasing the polymerization degree, or by adjusting the amount of oxygen in the high-speed dispersing device to perform oxygen-induced depolymerization of alkali cellulose.

If the high-speed dispersing device has a jacket, water may be passed through the jacket to reduce the heat generated by mixing the powdered pulp with the alkali metal hydroxide aqueous solution to control the internal temperature.

The obtained alkali cellulose may be used as it is in the next step, the etherification reaction. In order to make sure to obtain HPMC of which the interfacial viscous modulus, the interfacial elastic modulus and/or the ratio thereof fall into the above-mentioned range, it is preferable to prevent the polymerization degree of alkali cellulose from decreasing by adding the obtained alkali cellulose to a pressure container followed by subjecting the alkali cellulose to treatment to return to atmospheric pressure using nitrogen and/or decompression treatment.

Next, the etherification reaction using the obtained alkali cellulose, a methylating agent and a hydroxypropylating agent is conducted to obtain a crude product of HPMC.

Examples of methylating agents include methyl chloride. Examples of hydroxypropylating agents include propylene oxide.

The amounts of methylating agent and hydroxypropylating agent used may be determined appropriately depending on the DS of the methoxy groups and the MS of the hydroxypropoxy groups in HPMC. For example, relative to 1.0 part by mass of powdered pulp, the amount of methylating agent used is preferably in the range between 0.1 parts by mass and 3.0 parts by mass, and more preferably between 0.5 parts by mass and 2.0 parts by mass; and the amount of hydroxypropylating agent used is preferably in the range between 0.01 parts by mass and 3.0 parts by mass, and more preferably between 0.05 parts by mass and 1.0 parts by mass.

The reaction temperature for the etherification reaction is preferably from 40°C to 100°C. The reaction time for the etherification reaction is preferably from 1 hour to 5 hours.

The etherification reaction may be carried out in two or more stages by changing the temperature and/or time.

In the etherification reaction, a solvent for heat removal, such as dimethyl ether, may be used from the viewpoint of controllability of the reaction temperature.

The crude product of HPMC is then washed and dried to obtain HPMC.

Washing may be carried out using water. The temperature of water for washing is preferably from 85°C to 100°C.

Drying may be carried out using a dryer. Examples of dryer include, but are not particularly limited to, a conduction heat transfer type groove agitating dryer.

While the drying condition is not particularly limited, the condition that reduces the moisture content of HPMC to the extent that HPMC can be easily pulverized is preferred.

For example, the drying temperature is preferably from 50°C to 150°C.

The drying time is preferably from 1 hour to 15 hours.

The moisture content in HPMC after drying is not particularly limited, but for example, it is preferably from 0.8% by mass to 2.0% by mass. The moisture content in HPMC is determined according to "Loss on Drying Test" in Japanese Pharmacopoeia, 17th edition, as described in Examples below.

If desired, the obtained HPMC may be pulverized using a common pulverizer, such as a ball mill, a roller mill, and an impact pulverizer. Subsequently, the pulverized HPMC may be classified through a sieve to adjust to a desired particle size. The obtained HPMC may be simultaneously pulverizing and dried using a gas flow impact type pulverizer to obtain a powdered HPMC.

As long as solving the problems of the present invention is not prevented, the composition for aerosol cosmetics may contain, in addition to the above-mentioned HPMC, other HPMC different from the above-mentioned HPMC, such as HPMC with a ratio (E'/E") of interfacial elastic modulus (E') to interfacial viscous modulus (E") of less than 5.0 when measured with a tensiometer at 25°C using a 0.2% by mass aqueous solution. In addition, it is preferred that HPMC completely or substantially consists of the above-mentioned HPMC only.

(2) Organic solvent

The organic solvent is not particularly limited and may be any type of organic solvents normally employed as a component contained in aerosol cosmetics. Examples of organic solvent include lower alcohols with 2 to 6 carbons, polyhydric alcohols with 2 to 7 valences, hydrocarbon oils, ester oils, silicone oils, and fluorinated oils.

Specific examples of lower alcohols include ethanol, propanol, isopropanol and t-butanol. From the viewpoint of feeling of use, ethanol and isopropanol are preferred.

The carbon number in polyhydric alcohols is preferably from 2 to 7.

Specific examples of polyhydric alcohols include ethylene glycol, propylene glycol, glycerin, diglycerin, and 1,3-butylene glycol (BG; 1,3-butanediol). In terms of moisturizing properties and feeling of use, glycerin and 1,3-butanediol are preferred.

Specific examples of hydrocarbon oils include squalane, fluid paraffin, and isoparaffin.

Specific examples of ester oils include isopropyl myristate, cetyl ethylhexanoate, triethylhexanoin, cetyl isooctanoate, and isooctyl palmitate.

Specific examples of silicone oils include dimethyl silicone, phenylmethyl silicone, cyclopentasiloxane, and amodimethicone.

Specific examples of fluorinated oils include perfluoropolyethers.

The organic solvent may be used either individually or in combination of two or more of such organic solvents.

The content of organic solvent in the composition for aerosol cosmetics is not particularly limited and may be any amount normally employed in aerosol cosmetics. For example, relative to 100 parts by mass of HPMC, the content is preferably from 50 parts by mass to 500,000 parts by mass, more preferably from 500 parts by mass to 50,000 parts by mass, still more preferably from 1,000 parts by mass to 10,000 parts by mass, and still even more preferably from 2,000 parts by mass to 4,000 parts by mass.

(3) Water

Water is not particularly limited and may be any type of water commonly employed for the composition for aerosol cosmetics, including, for example, tap water and purified water.

The content of water in the composition for aerosol cosmetics is not particularly limited and may be any amount normally employed in aerosol cosmetics. For example, relative to 100 parts by mass of HPMC, the content is preferably from 1,000 parts by mass to 1,000,000 parts by mass, more preferably from 2,000 parts by mass to 500,000 parts by mass, still more preferably from 5,000 parts by mass to 50,000 parts by mass, and still even more preferably from 5,000 parts by mass to 25,000 parts by mass.

(4) Surfactant

In addition to HPMC with a ratio (E'/E") of interfacial elastic modulus (E') to interfacial viscous modulus (E") of 5.0 or higher when measured with a tensiometer at 25°C using a 0.2% by mass aqueous solution, an organic solvent and water, the composition for aerosol cosmetics may further contain other components such as a surfactant.

The surfactant is not particularly limited and may be any type of surfactant normally employed as a component in aerosol cosmetics. Examples of surfactants include anionic surfactants, cationic surfactants, ampholytic surfactants, and nonionic surfactants.

Specific examples of anionic surfactants include fatty acid salts such as sodium laurate (carbon number: 12), potassium laurate (carbon number: 12), potassium myristate (carbon number: 14), potassium palmitate (carbon number: 16), potassium stearate (carbon number: 18) and triethanolamine laurate (carbon number: 12); amino acid salts such as sodium cocoyl glutamate, sodium N-lauroyl-L-aspartate, sodium coconut fatty acid sarcosine and coconut fatty acid sarcosine triethanolamine; alkyl sulfate esters such as sodium lauryl sulfate and sodium stearyl sulfate; polyoxyethylene alkyl ether sulfates such as sodium laureth sulfate; phosphate esters such as sodium lauryl phosphate; sulfonates such as sodium dodecyl benzene sulfonate; and sulfosuccinates such as disodium laureth sulfosuccinate.

Specific examples of cationic surfactants include alkyl type quaternary ammonium salts such as stearyltrimethylammonium chloride and behentrimonium chloride; benzalkonium type quaternary ammonium salts such as benzalkonium chloride and benzethonium chloride.

Specific examples of ampholytic surfactants include alkylbetaines such as lauryl dimethylaminoacetate betaine and stearyl dimethylaminoacetate betaine; imidazoline type betaines such as disodium cocoamphodiacetate; betaine type ampholytic surfactants such as lauramidopropyl betaine, cocamidopropyl betaine and other alkylamidopropyl betaines; and amino acid type ampholytic surfactants such as sodium beta-laurylaminopropionate.

Specific examples of nonionic surfactants include sorbitan fatty acid esters such as sorbitan isostearate, sorbitan laurylate and sorbitan palmitate; polyoxyalkylene fatty acid esters such as polyethylene glycol monostearate ester and polyethylene glycol distearate ester; polyglycerin fatty acid esters such as decaglyceryl monostearate and decaglyceryl monolaurate; glycerin fatty acid esters such as glyceryl stearate, glyceryl isostearate, glyceryl oleate and glyceryl caprylate; polyoxyethylene glyceryl fatty acid esters such as polyethylene glycol-20 glyceryl isostearate, polyethylene glycol-60 glyceryl isostearate, polyethylene glycol-5 glyceryl stearate and polyethylene glycol-7 glyceryl coconut oil fatty acid ester; and polysorbates such as polysorbate 20, polysorbate 60 and polysorbate 80.

The surfactant may be used either individually or in combination of two or more surfactants. The surfactant may be either commercially available or obtained by extraction from a natural product or chemical synthesis.

The content of surfactant in the composition for aerosol cosmetics is not particularly limited and may be any amount normally employed in aerosol cosmetics. For example, from the viewpoint of foaming and emulsification, relative to 100 parts by mass of HPMC, the content is preferably from 10 parts by mass to 100,000 parts by mass, more preferably from 100 parts by mass to 10,000 parts by mass, and still more preferably from 500 parts by mass to 1,500 parts by mass.

(5) Additives

The composition for aerosol cosmetics may further contain, as other components, additives such as moisturizers, viscosity modifying agents, oily ingredients, pH adjusting agents, antioxidants, fragrances, preservative agents, whitening agents, pigments, UV absorbers, dyes, and disinfectants. Specific examples of additives are listed below, but the additives are not limited to those listed below.

Specific examples of moisturizers include hyaluronic acid, sodium hyaluronate, polyethylene glycol, mucopolysaccharide, urea, sorbitol, chondroitin sulfate, pyrrolidone carboxylic acid, sodium lactate and polyaspartic acid.

Specific examples of viscosity modifying agents include water-soluble polymers such as xanthane gum, guar gum, gellan gum, locust bean gum, carboxymethyl cellulose, hydroxyethyl cellulose, hydrophobized hydroxypropyl methyl cellulose, methylcellulose, cationized hydroxyethyl cellulose, carboxyvinyl polymer and polyvinyl alcohol.

Specific examples of oily ingredients include olive oil, macadamia nut oil, almond oil, camellia oil, shea oil, castor oil, coconut oil, beeswax, candelilla wax, carnauba wax, jojoba oil, lanolin, rice bran wax, lauric acid, myristic acid, palmitic acid, stearic acid, cetyl alcohol, stearyl alcohol, behenyl alcohol and microcrystalline wax.

Specific examples of pH adjusting agents include alkaline metal hydroxides such as sodium hydroxide and potassium hydroxide; alkaline earth metal hydroxides such as calcium hydroxide and magnesium hydroxide; ammonium carbonate, ammonia, ammonia water, trisodium phosphate, disodium hydrogen phosphate, dipotassium hydrogen phosphate; secondary alkylamines such as dimethylamine and diethylamine; tertiary alkylamines such as trimethylamine and triethylamine; monoethanolamine, isopropanolamine, diethanolamine, diisopropanolamine, triethanolamine, triisopropanolamine and polyethanolamine.

Specific examples of antioxidants include tocopherol, tocopherol acetate, ascorbic acid, butyl hydroxyanisole and dibutyl hydroxytoluene.

Specific examples of fragrances include natural fragrances and synthetic fragrances.

Specific examples of natural fragrances include rose oil, jasmine oil, lavender oil, ylang-ylang oil, peppermint oil, geranium oil, patchouli oil, sandalwood oil, cinnamon oil, lemon oil, orange oil and bergamot oil.

Specific examples of synthetic fragrances include limonene, β-caryophyhene, cis-3-hexenol, linalool, farnesol, β-phenylethyl alcohol, 2,6-nonadienal, citral, α-hexyl cinnamaldehyde, iota-carvone, cyclopentadecanone, linalyl acetate, γ-undecalactone and aurantiol.

Specific examples of preservative agents include methylparaben, ethylparaben, propylparaben, butylparaben and phenoxyethanol.

Specific examples of whitening agents include arbutin, α-arbutin, ascorbic acid; ascorbic acid fatty acid esters such as sodium ascorbate phosphate, magnesium ascorbate phosphate and tetraisopalmitate ascorbate; kojic acid, ellagic acid and tranexamic acid; and the derivatives thereof.

Specific examples of pigments include titanium dioxide, zinc oxide, barium sulfate, zinc oxide coated or compounded with silicic anhydride, iron oxide (bengara), iron titanate, γ-iron oxide, iron yellow oxide, ochre, black iron oxide, carbon black and low-order titanium dioxide.

Specific examples of UV absorbers include octyl methoxysilicate, octyl dimethoxybenzylidenedioxoimidazolidine propionate, hexyl diethylaminohydroxybenzoyl benzoate, t-butyl methoxydibenzoylmethane, octyl triazone and 2-ethylhexyl paramethoxycinnamic acid.

Specific examples of dyes include acid dyes, nitro dyes, disperse dyes, basic dyes and oxidative dye intermediates.

Specific examples of disinfectants include benzalkonium chloride, triclosan and hinokitiol.

The additives may be used either individually or in combination of two or more of such additives. The additives may be either commercially available or obtained by extraction from a natural product or chemical synthesis.

The content of additives in the composition for aerosol cosmetics is not particularly limited and may be any amount normally employed in aerosol cosmetics. For example, relative to 100 parts by mass of HPMC, the content is preferably from 50 parts by mass to 50,000 parts by mass, more preferably from 100 parts by mass to 5,000 parts by mass, and still more preferably from 400 parts by mass to 1,500 parts by mass.

(6) Composition for aerosol cosmetics

The composition for aerosol cosmetics according to one embodiment of the present invention contains HPMC, which has a ratio of interfacial elastic modulus (E') to interfacial viscous modulus (E") of 5.0 or higher when measured with a tensiometer at 25°C using a 0.2% by mass aqueous solution, an organic solvent and water so that the composition can have improved dispensability, and can stably make a foam with greater foam quality. As long as the composition for aerosol cosmetics according to one embodiment of the present invention has such properties, other properties of the composition are not particularly limited.

However, the viscosity of the composition for aerosol cosmetics tends to affect the dispensability and the condition of foam after dispensing. The viscosity at 20°C of the composition for aerosol cosmetics is preferably from 10.0 mPa·s to 3000.0 mPa·s, more preferably from 10.0 mPa·s to 2000.0 mPa·s, and still more preferably from 10.0 mPa·s to 1000.0 mPa·s in terms of dispensability, and handling ability during working such as preparation and filling.

The viscosity at 20°C of the composition for aerosol cosmetics is a value measured using a rheometer, as described in Examples below.

The composition for aerosol cosmetics can be produced, for example, by mixing HPMC, an organic solvent and water, and if necessary, a surfactant and/or additives required to obtain the desired cosmetics.

The way of mixing each component with each other in the composition for aerosol cosmetics is not particularly limited. For example, from the viewpoint of simplification of the process and solubilization of HPMC, a method including the following steps is preferred: mixing an organic solvent and water, and, if necessary, a surfactant and/or additives with each other by an agitating device such as a three-one motor agitating device; warming the resulting mixture up to 70°C to 80°C; adding HPMC to the warmed mixture while stirring to obtain a uniform dispersion solution; and then cooling the dispersion solution to 0°C to 5°C.

The composition for aerosol cosmetics is used in various applications, and such applications are not particularly limited as long as the composition is used in cosmetic applications. Examples of such applications include skin cleansers, cleansing products, serums, skin toners, skin milks, hair treatments, hair styling products, sunscreens and body milks.

Aerosol cosmetics

According to another aspect of the present invention, there are provided aerosol cosmetics. The aerosol cosmetics according to one embodiment of the present invention contains the composition for aerosol cosmetics according to one embodiment of the present invention and a propellant in an aerosol container.

The aerosol cosmetics can be produced, for example, by filling a propellant into an aerosol container containing the composition for aerosol cosmetics. The aerosol cosmetics result in a foaming cosmetic product by dispensing the composition for aerosol cosmetics from the aerosol container when used.

The aerosol container is not particularly limited as long as the aerosol container is any pressure-resistant container equipped with a mechanism that allows the contents to be dispersed by pushing down a dispersing button containing an actuator. Examples of aerosol container include AEW cans, AL cans, double-layered containers and plastic containers.

The propellant is not particularly limited as long as the propellant is a pressurized gas normally employed in producing aerosol cosmetics. Examples of propellant include a liquefied petroleum gas (LPG) such as dimethyl ether, isopentane and fluorocarbon; carbon dioxide gas, nitrogen gas, and nitrous oxide gas.

The internal pressure in the aerosol container is not particularly limited as long as the internal pressure is any internal pressure normally used in aerosol cosmetics. The internal pressure is, from the viewpoint of dispensability and foaming ability, preferably in the range between 0.4 MPa and 1.0 MPa at 25°C.

When using the aerosol cosmetics, it is preferable to shake the container several times, dozens of times or hundreds of times, specifically 10 times to 100 times, from the perspective of achieving uniformity of the composition for aerosol cosmetics in the container.

The aerosol cosmetics according to one embodiment of the present invention can have a more improved dispensability and can stably make a foam with a better quality after dispersing as compared to the aerosol cosmetics produced under the same condition as the aerosol cosmetics according to one embodiment of the present invention (hereinafter referred to as "comparative aerosol cosmetics"), except that the comparative aerosol cosmetics contain HPMC with a ratio of interfacial elastic modulus (E') to interfacial viscous modulus (E") of less than 5.0 when measured with a tensiometer at 25°C using a 0.2% by mass aqueous solution, instead of HPMC with a ratio of 5.0 or higher.

The dispensability and foam condition of the aerosol cosmetics according to one embodiment of the present invention can be evaluated by the section "Dispensability", "Foam hardness", "Foam height", and/or "Bouncy foam feeling" as described in Examples below.

For example, the dispensability of the aerosol cosmetics according to one embodiment of the present invention is preferably evaluated as the rating "++" or "+", and more preferably as the rating "++". As for the foam condition of the aerosol cosmetics according to one embodiment of the present invention, the foam hardness is preferably evaluated to be larger than that of the comparative aerosol cosmetics; the foam height is preferably evaluated to be higher than that of the comparative aerosol cosmetics; and/or the bouncy foam feeling is preferably evaluated as the rating "++" or "+", and more preferably the rating"++".

One preferred embodiment of the present invention is aerosol cosmetics with a dispensability evaluated as the rating "++"; and a bouncy foam feeling evaluated as the rating "++ or "+". Another preferred embodiment of the present invention is aerosol cosmetics in which the foam hardness and foam height are evaluated to be larger and higher than the comparative aerosol cosmetics, respectively.

Other aspects of invention

Another aspect of the present invention relates to a method of producing a composition for aerosol cosmetics, including the step of mixing a solution containing an organic solvent and water with hydroxypropyl methylcellulose to obtain the composition for aerosol cosmetics, wherein a ratio (E'/E") of interfacial elastic modulus (E') to interfacial viscous modulus (E") of the hydroxypropyl methylcellulose is equal to or higher than 5.0 when measured with a tensiometer at 25°C using a 0.2% by mass aqueous solution.

Another aspect of the present invention relates to a method of producing aerosol cosmetics, including the steps of mixing a solution containing an organic solvent and water with hydroxypropyl methylcellulose to obtain the composition for aerosol cosmetics; and filling the composition for aerosol cosmetics and a propellant in an aerosol container to obtain the aerosol cosmetics, wherein a ratio (E'/E") of interfacial elastic modulus (E') to interfacial viscous modulus (E") of the hydroxypropyl methylcellulose is equal to or higher than 5.0 when measured with a tensiometer at 25°C using a 0.2% by mass aqueous solution.

Examples

While the present invention will now be described in further detail with reference to Synthesis Examples, Examples and Comparative Examples, the present invention is not limited to these Synthesis Examples and Examples.

Preparation of powdered pulp, and measurement of its physical properties Three commercially available high-purity dissolving pulps from wood were pulverized to obtain three types of powdered pulps (powdered pulps A, B and C) with different viscosities.

The physical properties of the obtained powdered pulps A, B and C were measured by the following method. As a result, the powdered pulps A, B and C had D50 of 150 $\mu$m, and moisture content of 4.0% by mass. Furthermore, the viscosity of powdered pulp A was 800 ml/g; the viscosity of powdered pulp B was 1,500 ml/g; and the viscosity of powdered pulp C was 1,700 ml/g.

Weight average particle size (D50) of powdered pulp

Test sieves with different mesh sizes according to JIS Z8801 were mounted on an RO-TAP sieve shaker. The powdered pulp was then loaded onto the top sieve and subjected to oscillation or tapping to sieve the powdered pulp. The weight of pulp on each sieve and the weight of pulp below the sieve were determined to prepare a weight-based distribution, which in turn was used to regard a size of 50% of integration value (cumulative 50% size) as an average particle size (D50).

Moisture content of powdered pulp

The moisture content of powdered pulp was calculated by using the following equation by using the dry matter content determined according to the test method of pulp-dry matter content as described in JIS P8203: 1998.

$$\text{Moisture content (\%) = 100 - Dry matter content (\%)}$$

In the above equation, the dry matter content is a ratio (%) of the mass of powdered pulp in the case where the sample is dried at 105$\pm$2°C and reaches a constant mass relative to the mass of powdered pulp before drying.

Viscosity of powdered pulp

The viscosity was measured according to the viscosity measurement method in ISO 5351. In addition, the value corresponds to intrinsic viscosity.

Preparation of HPMC

Synthesis Example 1: Preparation of HPMC-1

The powdered pulp A (10.2 kg; viscosity: 800 ml/g) at 36 kg/hr using an auto feeder ("FS-Q2-S"; manufactured by Funken Powtechs), and the sodium hydroxide aqueous solution (24.5 kg; 49% by mass; 40°C) at 86.5 kg/hr using a mohno-pump ("Model N"; manufactured by Heishin) were continuously and simultaneously fed into a flow jet mixer (high-speed dispersing device; "MW-F-300-X"; manufactured by Funken Powtechs) and then mixed with each other to produce an alkali cellulose.

The peripheral speed on the agitator blade in the high-speed dispersing device was set to 7 m/s (rotation speed 446 rpm), and the disk type agitator blade (300 mm in diameter) with scraping blades was employed as the agitator blade. The holding time in the mixing container of the high-speed dispersing device was 2 seconds.

The feeding opening of the sodium hydroxide aqueous solution was provided at a different location near the feeding opening of the powdered pulp.

The obtained alkali cellulose (22.7 kg) was placed in a pro-shear type pressure container with internal stirring means of which the internal volume is 100 liters, and the pressure was reduced to -97 kPa. The pressure was then recovered to atmospheric pressure with nitrogen followed by being reduced to -97 kPa again. To the pressure container, 2.8 kg of dimethyl ether was added, and 14.3 kg of methyl chloride and 3.5 kg of propylene oxide were then added. The reaction with the pressure container was carried out at an internal temperature of 66°C for 2 hours. Then, the pressure container was heated up and the reaction was carried out at an internal temperature of 90°C for 30 minutes to obtain a crude product of HPMC.

The obtained crude product of HPMC was washed with hot water at 90°C, then dried at 80°C for 10 hours, and pulverized by a pulverizing device to obtain HPMC-1 in its powdered form (moisture content: 1% by mass).

For the resulting HPMC-1, the viscosity at 20°C of 2.0% by mass aqueous solution, the DS of the methoxy groups, the MS of the hydroxypropoxy groups, the interfacial viscous modulus (E") of 0.2% by mass aqueous solution at 25°C, and the interfacial elastic modulus (E') of 0.2% by mass aqueous solution at 25°C were measured, and the ratio (E'/E") of interfacial elastic modulus (E') to interfacial viscous modulus (E") was calculated.

Synthesis Example 2: Preparation of HPMC-2

HPMC was produced by the same method as in Synthesis Example 1 to obtain HPMC-2 (moisture content: 1% by mass), except that the powdered pulp B (viscosity: 1,500 ml/g) was used, and the peripheral speed on the agitator blade in the high-speed dispersing device was set to 5 m/s (rotation speed: 318 rpm).

For the resulting HPMC-2, the viscosity at 20°C of 2.0% by mass aqueous solution, the DS of the methoxy groups, the MS of the hydroxypropoxy groups, the interfacial viscous modulus (E") of 0.2% by mass aqueous solution at 25°C, and the interfacial elastic modulus (E') of 0.2% by mass aqueous solution at 25°C were measured, and the ratio (E'/E") of interfacial elastic modulus (E') to interfacial viscous modulus (E") was calculated.

Synthesis Example 3: Preparation of HPMC-3

The powdered pulp C (10.2 kg; viscosity: 1,700 ml/g) at 36 kg/hr using an auto feeder ("FS-Q2-S"; manufactured by Funken Powtechs), and the sodium hydroxide aqueous solution (19.6 kg; 49% by mass; 40°C) at 69.2 kg/hr using a mohno-pump ("Model N"; manufactured by Heishin) were continuously and simultaneously fed into a flow jet mixer (high-speed dispersing device; "MW-F-300-X"; manufactured by Funken Powtechs) and then mixed to produce an alkali cellulose.

The peripheral speed on the agitator blade in the high-speed dispersing device was set to 14 m/s (rotation speed 891 rpm), and the disk type agitator blade (300 mm in diameter) with scraping blades was employed as the agitator blades. The holding time in the mixing container of the high-speed dispersing device was 2 seconds.

The feeding opening of the sodium hydroxide aqueous solution was provided at a different location near the feeding opening of the powdered pulp.

The obtained alkali cellulose (19.1 kg) was placed in a plowshare type pressure container with internal stirring means of which the internal volume is 100 liters, and the pressure was reduced to -97 kPa. The pressure was then recovered to atmospheric pressure with nitrogen followed by being reduced to -97 kPa again. To the pressure container, 2.8 kg of dimethyl ether was added, and 11.4 kg of methyl chloride and 1.2 kg of propylene oxide were then added. The reaction with the pressure container was carried out at an internal temperature of 66°C for 2 hours. Then, the pressure container was heated up and the reaction was carried out at an internal temperature of 90°C for 30 minutes to obtain a crude product of HPMC.

The obtained crude product of HPMC was washed with hot water at 90°C, then dried at 80°C for 10 hours, and pulverized by a pulverizing device to obtain HPMC-3 in the powdered form (moisture content: 1% by mass).

For the resulting HPMC-3, the viscosity at 20°C of 2.0% by mass aqueous solution, the DS of the methoxy groups, the MS of the hydroxypropoxy groups, the interfacial viscous modulus (E") of 0.2% by mass aqueous solution at 25°C, and the interfacial elastic modulus (E') of 0.2% by mass aqueous solution at 25°C were measured, and the ratio (E'/E") of interfacial elastic modulus (E') to interfacial viscous modulus (E") was calculated.

Synthesis Example 4: Preparation of HPMC-4

HPMC was produced by the same method as in Synthesis Example 1 to obtain HPMC-4 (moisture content: 1% by mass), except that the peripheral speed on the agitator blade in the high-speed dispersing device was set to 2 m/s (rotation speed: 127 rpm).

For the resulting HPMC-4, the viscosity at 20°C of 2.0% by mass aqueous solution, the DS of the methoxy groups, the MS of the hydroxypropoxy groups, the interfacial viscous modulus (E") of 0.2% by mass aqueous solution at 25°C, and the interfacial elastic modulus (E') of 0.2% by mass aqueous solution at 25°C were measured, and the ratio (E'/E") of interfacial elastic modulus (E') to interfacial viscous modulus (E") was calculated.

Measurements of physical properties of HPMC

The physical properties of HPMC were measured for HPMC-1 to -4 prepared in Synthesis Examples 1 to 4. The method for measuring each property is shown below. The result of each measurement is shown in Table 1.

Interfacial viscous modulus (E") and interfacial elastic modulus (E')

Using a tensiometer ("Tracker S" manufactured by Teclis), the values at 25°C were measured for a 0.2% by mass aqueous solution of HPMC as a sample solution.

An amount of HPMC corresponding to 0.6 g of the dried product adjusted for the moisture content was accurately weighed in a jar (diameter: 65 mm, height: 120 mm, volume: 350 ml). To the jar, hot water at 98°C was added to amount to 300.0 g. The jar was capped, and the mixture was stirred for 20 minutes using a stirrer at 350 rpm to 500 rpm until a uniform dispersion was obtained. Subsequently, the dispersion was stirred for 40 minutes in a water bath at 0°C to 5°C to obtain a 0.2% by mass aqueous solution of HPMC as a sample solution.

An 18-gauge needle as a syringe for the rising drop measurement was attached to the tensiometer. The sample measurement unit of the tensiometer was conditioned to 25°C in advance. The prepared 0.2% by mass aqueous solution of HPMC was poured into a measurement cup (diameter: 25 mm, height: 66 mm, rectangular parallelepiped glass container) to the marked line (25 ml), and measurements were started under the following conditions: While the sample measurement unit of the tensiometer was kept constant at 25°C, data were collected over a period of 100 seconds or longer to obtain a sine wave. With the calculation frequency of 100 seconds, the interfacial viscous modulus (E") and the interfacial elastic modulus (E') were determined, and the ratio E'/E" was calculated.

Measurement conditions for tensiometer:

Period: 10 sec

Active cycles: 5 cycles

Blank cycles: 5 cycles

Injected air: air with a volume of 16.6 ± 0.5 mm2

Variation intensity (Amplitude): 0.7 mm2

Viscosity

A 2% by mass aqueous solution of HPMC was prepared in a similar manner to the method of preparing a 0.2% by mass aqueous solution of HPMC, and the viscosity at 20°C of 2% by mass aqueous solution of HPMC was measured by using a single cylinder type rotational viscometer according to "2. Method II Viscosity measurement by rotational viscometer" in the Viscosity Determination in General Tests described in Japanese Pharmacopoeia, 17th edition.

Degree of substitution of methoxy groups (DS) and molar substitution (MS) of hydroxypropoxy groups

MS and DS were converted from the results measured according to the quantification method of "Hypromellose" in Japanese Pharmacopoeia, 17th Edition.

Moisture content

The moisture content was measured according to the Loss on Drying Test in General Tests described in Japanese Pharmacopoeia, 17th edition.

Table 1

| | Ratio of interfacial elastic modulus (E') to interfacial viscous modulus (E") (E'/E") (-) | Interfacial viscous modulus (E") (mN/m) | Interfacial elastic modulus (E') (mN/m) | Viscosity (mPa·s) | DS (-) | MS (-) |
|---|---|---|---|---|---|---|
| HPMC-1 | 6.0 | 17.8 | 108.3 | 5210 | 1.89 | 0.24 |
| HPMC-2 | 7.5 | 15.7 | 118.4 | 9500 | 1.89 | 0.24 |
| HPMC-3 | 8.6 | 24.1 | 206.3 | 15000 | 1.78 | 0.16 |
| HPMC-4 | 3.1 | 13.0 | 40.3 | 5040 | 1.89 | 0.24 |
| * In table, HPMC represents hydroxypropyl methylcellulose. | | | | | | |

Production of composition for aerosol cosmetics, and preparation of aerosol cosmetics

HPMC used as an employed material was HPMC-1 to -4 prepared in Synthesis Examples 1 to 4. For organic solvents, 1,3-butanediol (manufactured by Wako Pure Chemical) and glycerin (manufactured by Kishida Chemical) were used. Tap water was used for water. For additives, polyethylene glycol ("Polyethylene glycol 400"; manufactured by Wako Pure Chemical) was used as a moisturizer.

For surfactants, potassium myristate aqueous solution, potassium laurate aqueous solution, potassium palmitate aqueous solution, and potassium stearate aqueous solution were used. The above surfactant aqueous solutions were prepared by adding each of myristic acid (manufactured by Wako Pure Chemical), lauric acid (manufactured by Wako Pure Chemical), palmitic acid (manufactured by Wako Pure Chemical) and stearic acid (manufactured by Kishida Chemical) to a potassium hydroxide aqueous solution containing potassium hydroxide (manufactured by Wako Pure Chemical) in the equal molar amounts, and then mixing them. PEG monostearate (polyethylene glycol monostearate (40 E.O.), manufactured by Nikko Chemicals) was used as another surfactant.

Example 1

To a jar (diameter: 65 mm, height: 120 mm, volume: 350 ml), 11.7 g of potassium myristate aqueous solution (containing 7.5 g of potassium myristate), 25 g of 1,3-butanediol, and 62.4 g of hot water at 98°C were accurately weighed and added. Then, the jar was placed in a hot water bath heated to 70°C to 80°C, and the solution in the jar was stirred at 200 rpm to 350 rpm for 30 minutes using an agitator ("MAZELA-Z"; manufactured by Tokyo Rikakikai) such that the solution became a uniform dispersion solution.

While keeping the jar warmed at 70°C to 80°C, 0.9 g of HPMC-1 was added to the solution in the jar, and the mixture in the jar was stirred at 350 rpm to 500 rpm for 10 minutes until the pulverized HPMC was uniformly dispersed. Then, HPMC was dissolved by stirring the mixture in a water bath at 0°C to 5°C for 40 minutes to produce a composition for aerosol cosmetics.

The composition for aerosol cosmetics was then transferred to an espuma container ("Advanced Dispenser S," manufactured by Nippon Tansan Gas), and nitrogen gas was filled until the internal pressure of the container reached to 0.6 MPa, resulting in aerosol cosmetics. The espuma container was manually shaken for 20 times, and the composition was then dispensed at 25°C from the nozzle to prepare foamed aerosol cosmetics.

Example 2

For Example 2, the composition for aerosol cosmetics was produced, and the aerosol cosmetics were then prepared by the same method as in Example 1, except that 0.75 g of HPMC-2 and 62.55 g of hot water at 98°C were used in place of 0.9 g of HPMC-1 and 62.4 g of hot water at 98°C, respectively.

Example 3

For Example 3, the composition for aerosol cosmetics was produced, and the aerosol cosmetics were then prepared by the same method as in Example 1, except that 0.75 g of HPMC-3 and 62.55 g of hot water at 98°C were used in place of 0.9 g of HPMC-1 and 62.4 g of hot water at 98°C, respectively.

Comparative Example 1

For Comparative Example 1, the composition for aerosol cosmetics was produced, and the aerosol cosmetics were then prepared by the same method as in Example 1, except that 0.9 g of HPMC-4 was used in place of 0.9 g of HPMC-1.

Example 4

To a jar (diameter: 65 mm, height: 120 mm, volume: 350 ml), a mixture of potassium myristate, potassium laurate, potassium palmitate and potassium stearate with water (wherein 7.5 g of the mixture contains 2.1 g of potassium myristate, 0.49 g of potassium laurate, 0.37 g of potassium palmitate, and 0.25 g of potassium stearate, respectively), 10 g of glycerin, 2.0 g of 1,3-butanediol, 5.0 g of polyethylene glycol, 1.0 g of PEG monostearate, and 78.3 g of hot water at 98°C were accurately weighed and added. Then, the jar was placed in a hot water bath heated at 70°C to 80°C, and the solution in the jar was stirred at 200 rpm to 350 rpm for 30 minutes using an agitator ("MAZELA-Z"; manufactured by Tokyo Rikakikai) such that the solution became a uniform dispersion solution.
While keeping the jar warmed at 70°C to 80°C, 0.5 g of HPMC-1 was added to the solution in the jar, and the mixture in the jar was stirred at 350 rpm to 500 rpm for 10 minutes until the pulverized HPMC was uniformly dispersed. Then, HPMC was dissolved by stirring the mixture in a water bath at 0°C to 5°C for 40 minutes to produce a composition for aerosol cosmetics.
The composition for aerosol cosmetics was then transferred to an espuma container ("Advanced Dispenser S," manufactured by Nippon Tansan Gas), and nitrogen gas was filled until the internal pressure of the container reached to 0.5 MPa, resulting in aerosol cosmetics. The espuma container was manually shaken for 20 times, and the composition was then dispensed at 25°C from the nozzle to prepare foamed aerosol cosmetics.

Example 5

For Example 5, the composition for aerosol cosmetics was produced, and the aerosol cosmetics were then prepared by the same method as in Example 4, except that 0.4 g of HPMC-2 and 78.4 g of hot water at 98°C were used in place of 0.5 g of HPMC-1 and 78.3 g of hot water at 98°C, respectively.

Example 6

For Example 6, the composition for aerosol cosmetics was produced, and the aerosol cosmetics were then prepared by the same method as in Example 4, except that 0.4 g of HPMC-3 and 78.4 g of hot water at 98°C were used in place of 0.5 g of HPMC-1 and 78.3 g of hot water at 98°C, respectively.

Comparative Example 2

For Comparative Example 2, the composition for aerosol cosmetics was produced, and the aerosol cosmetics were then prepared by the same method as in Example 4, except that 0.5 g of HPMC-4 was used in place of 0.5 g of HPMC-1.

Measurement of physical properties of composition for aerosol cosmetic

The viscosities of the compositions for aerosol cosmetics prepared in Examples 1 to 6 and Comparative Examples 1 to 2 were measured. The method for measuring the viscosity is shown below. The results of viscosity measurement are shown in Tables 2 and 3.

Viscosity

The sample measurement unit of a rheometer ("MCR-301," manufactured by Anton Paar) was pre-conditioned to 20°C, and the composition for aerosol cosmetics after preparation was poured into a CC27 measurement cup (a cylindrical aluminum container with a diameter of 30 mm and a height of 80 mm) to the marked line (25 ml). Measurements were then started with a shear rate of 50 1/s. The measurement unit was kept constant at 20°C, and data were collected one per minute. The shear viscosity measured 10 minutes after the start of measurement was assigned to be the viscosity at 20°C of the composition for aerosol cosmetics.

Measurements of physical properties of aerosol cosmetics

As for the properties of the aerosol cosmetics prepared in Examples 1 to 6 and Comparative Examples 1 to 2, the foam hardness and foam height were measured. The methods for measuring the foam hardness and foam height are shown below. The results of such measurements are shown in Tables 2 and 3.

Foam hardness

The foam hardness is a repulsive force of foam when applying a load to the foam and is an index of the elasticity and bouncy foam feeling of foam. The smaller the foam hardness is, the smaller the elasticity of foam is, and the less the foam has a bouncy foam feeling.
The foam hardness was measured by using "Curdmeter MAX ME-500", manufactured by Aska Equipments. Aerosol cosmetics immediately after dispensed was filled in a plastic container (volume: 25 ml; height: 58.0 mm) without any gaps, and the top was then scraped off. The foam hardness was measured as a hardness of foam immediately after dispensed.
The measurement conditions are as follows.

Measurement jig: φ16mm

Initial spring load: 100 g

Ascending speed of sample stage: 0.36 cm/sec

Sample volume: 25 ml

Foam height

The foam height indicates the degree of disappearance of foam over a certain period and is an index of foam stability. The lower the foam height is, the sooner the foam disappears, and the worse the foam holding is.
Aerosol cosmetics dispensed from an espuma container were filled into a plastic container (volume: 25 ml, height: 62.3 mm) without any gaps, and the top was then scraped off. The container was allowed to leave for stand for 15 minutes at room temperature, and the foam height was measured using a ruler.

Sensory evaluation of aerosol cosmetics

In the sensory evaluation, the aerosol cosmetics prepared in Examples 1 to 6 and Comparative Examples 1 to 2 were evaluated by five panelists in view of dispensability and bouncy foam feeling. Five panelists were asked about whether the aerosol cosmetics are good for each evaluation item. Based on the number of panelists whose answers were good, the aerosol cosmetics were evaluated according to the following evaluation criteria.

Dispensability

The dispensability of foam was evaluated as the evaluation "good" if through visual observation, the dispensing difficulty due to the sogginess, chipping and/or clogginess of the foam was not observed, and the fine foam was smoothly dispensed.

Scoring standard

++: 5 out of 5 panelists answered good;
+: 3 to 4 out of 5 panelists answered good;
+-: 1 to 2 out of 5 panelists answered good; and

-: nobody out of 5 panelists answered good.

Bouncy foam feeling

The bouncy foam feeling of foam was evaluated as the evaluation "good" if the foam did not disappear immediately and the elasticity was felt through the whole foam in the case where the aerosol cosmetics were dispensed from the espuma container, and the resulting foam of which the diameter of the bottom was about 5 cm was placed on the palm of the left hand and then kneaded with the palm of the right hand.

Scoring standard

++: 5 out of 5 panelists answered good;
+: 3 to 4 out of 5 panelists answered good;
+-: 1 to 2 out of 5 panelists answered good; and
-: nobody out of 5 panelists answered good.

Table 2

| | Composition for aerosol cosmetics | | | | | Evaluation of characteristics of aerosol cosmetics | | Sensory evaluation of aerosol cosmetics | |
|---|---|---|---|---|---|---|---|---|---|
| | HPMC (% by mass) | Organic solvent (% by mass) | Water (% by mass) | Surfacntant (% by mass) | Viscosity (mPa ▪ s) | Foam hardness (g) | Foam height (mm) | Dispensability | Fluffy feeling |
| Example 1 | HPMC-1 [0.9] | BG [25] | Purified water [66.6] | Potassium myristate [7.5] | 651 | 2.2 | 42 | ++ | + |
| Example 2 | HPMC-2 [0.75] | BG [25] | Purified water [66.75] | Potassium myristate [7.5] | 566 | 2.3 | 42 | ++ | + |
| Example 3 | HPMC-3 [0.75] | BG [25] | Purified water [66.75] | Potassium myristate [7.5] | 563 | 2.4 | 42.8 | ++ | ++ |
| Comparative Example 1 | HPMC-4 [0.9] | BG [25] | Purified water [66.6] | Potassium myristate [7.5] | 527 | 1.8 | 35 | + | +- |
| * In table, BG represents 1,3-butanediol. | | | | | | | | | |

Table 3

| | Composition for aerosol cosmetics | | | | | | | | | | Evaluation of characteristics of aerosol cosmetics | | Sensory evaluation of aerosol cosmetics | |
|---|---|---|---|---|---|---|---|---|---|---|---|---|---|---|
| | HPMC (% by mass) | Organic solvent (% by mass) | | Water (wt%) | Surfactant (% by mass) | | | | | Additives (% by mass) | Viscosity (mPa▪s) | Foam hardness (g) | Foam height (mm) | Dispensability | Fluffy feeling |
| Example 4 | HPMC-1 [0.5] | Glycerin [10] | BG [2.0] | Water [78.3] | Potassium myristate [2.1] | Potassium laurate [0.49] | Potassium palmitate [0.37] | Potassium stearate [0.25] | PEG monostearate [1.0] | PEG [5.0] | 120 | 3.8 | 48 | ++ | + |
| Example 5 | HPMC-2 [0.4] | Glycerin [10] | BG [2.0] | Water [78.4] | Potassium myristate [2.1] | Potassium laurate [0.49] | Potassium palmitate [0.37] | Potassium stearate [0.25] | PEG monostearate [1.0] | PEG [5.0] | 103 | 4.9 | 48 | ++ | + |
| Example 6 | HPMC-3 [0.4] | Glycerin [10] | BG [2.0] | Water [78.4] | Potassium myristate [2.1] | Potassium laurate [0.49] | Potassium palmitate [0.37] | Potassium stearate [0.25] | PEG monostearate [1.0] | PEG [5.0] | 126 | 5.3 | 51 | ++ | ++ |
| Comparative Example 2 | HPMC-4 [0.5] | Glycerin [10] | BG [2.0] | Water [78.3] | Potassium myristate [2.1] | Potassium laurate [0.49] | Potassium palmitate [0.37] | Potassium stearate [0.25] | PEG monostearate [1.0] | PEG [5.0] | 100 | 3.3 | 40 | + | +- |
| * In table, PEG represents polyethylene glycol. | | | | | | | | | | | | | | | |

Results of evaluation of aerosol cosmetics

From the results of Examples 1 to 6 and Comparative Examples 1 to 2, the aerosol cosmetics prepared using the compositions for aerosol cosmetics containing HPMC-1 to 3 (Examples 1 to 6) had more favorable foam holding (foam height) and foam hardness, and was better in terms of sensory evaluation such as foam dispensability and bouncy foam feeling, as compared to the aerosol cosmetics prepared using the compositions for aerosol cosmetics containing HPMC-4 (Comparative Examples 1 to 2).

These results suggest that the use of HPMC with the ratio E'/E" of 5.0 or higher allows foam films to be stably formed and thereby improving foam stability and suppressing undesired decreases in foam holding (foam height) and foam hardness. From the results of Examples 4 to 6, it was found that the higher the ratio E'/E" of HPMC, the more favorable the foam holding (foam height) and foam hardness of the resulting aerosol cosmetics. In other words, it was found that there was a positive correlation between the ratio E'/E" in HPMC and the foam holding (foam height) and foam hardness achieved by the resulting aerosol cosmetics.

Furthermore, the use of HPMC with the interfacial viscous modulus of 15.0 mN/m or higher allowed foam films to be stably formed while causing moderate foaming in the course of dispensing the aerosol cosmetics. It was assumed that such aerosol cosmetics could form a stronger foam that is difficult to be defoamed and could provide a foam with an excellent foam holding (foam height) and foam hardness.

The favorable foam height and foam hardness caused the excellent results of sensory evaluation in terms of foam dispensability and bouncy foam feeling, resulting in good foam properties.

From the results of Examples 4 to 6 and Comparative Example 2, it was confirmed that even when several types of organic solvents and surfactants were used in combination, the aerosol cosmetics were not affected by them, and had favorable foam properties.

Industrial applicability

According to the composition for aerosol cosmetics according to one embodiment of the present invention, it is possible to produce aerosol cosmetics that have improved foam dispensability and render a foam condition obtained by foaming excellent. The aerosol cosmetics according to one embodiment of the present invention can be widely distributed. The composition for aerosol cosmetics and the aerosol cosmetics according to one embodiment of the present invention can be produced and used on industrial scales.

**Claims**

1. A composition for aerosol cosmetics, comprising hydroxypropyl methylcellulose, an organic solvent and water, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution.

2. The composition according to claim 1, wherein the ratio of interfacial elastic modulus (E') to interfacial viscous modulus (E") of the hydroxypropyl methylcellulose ranges from 5.0 to 17.0, preferably from 5.5 to 9.0, more preferably from 5.7 to 8.6.

3. The composition for aerosol cosmetics according to any one of claims 1 to 2, wherein the interfacial viscous modulus (E") of the hydroxypropyl methylcellulose is equal to or higher than 15.0 mN/m, preferably 15.0 mN/m to 40.0 mN/m, more preferably 15.3 mN/m to 35.0 mN/m, even more preferably from 15.5 mN/m to 28.0 mN/m, when measured with a tensiometer at 25°C using a 0.2% by mass aqueous solution.

4. The composition for aerosol cosmetics according to any one of claims 1 to 3, wherein the interfacial elastic modulus (E') of the hydroxypropyl methylcellulose is in a range between 80.0 mN/m and 250.0 mN/m, preferably 85.0 mN/m to 230.0 mN/m, more preferably 90.0 mN/m to 210.0 mN/m, even more preferably 100.0 mN/m to 208.0 mN/m, when measured with a tensiometer at 25°C using a 0.2% by mass aqueous solution.

5. The composition for aerosol cosmetics according to any one of claims 1 to 4, wherein the viscosity of the hydroxypropyl methylcellulose is in a range between 1,000 mPa·s and 100,000 mPa·s, preferably 1,000 mPa·s to 75,000 mPa·s, more preferably 3,500 mPa·s to 20,000 mPa·s, when measured with a viscometer at 20°C using a 2% by mass aqueous solution.

6. The composition for aerosol cosmetics according to any one of claims 1 to 5, wherein the degree of substitution of methoxy groups in hydroxypropyl methylcellulose is 1.00 to 2.20, preferably 1.50 to 2.00, more preferably 1.60 to 1.95.

7. The composition for aerosol cosmetics according to any one of the claims 1 to 6, wherein the molar substitution of hydroxypropyl methylcellulose is 0.10 to 0.60, preferably 0.15 to 0.35.

8. The composition for aerosol cosmetics according to any one of claims 1 to 7, wherein the organic solvent is used individually or in combination of two or more organic solvents.

9. The composition for aerosol cosmetics according to any one of claims 1 to 8, wherein the organic solvent is selected from lower alcohols with 2 to 6 carbons, polyhydric alcohols with 2 to 7 valences, hydrocarbon oils, ester oils, silicone oils and/or fluorinated oils, preferably from ethanol, isopropanol, glycerine and/or 1,3-butanediol.

10. The composition for aerosol cosmetics according to any one of claims 1 to 9, further comprising a surfactant.

11. The composition for aerosol cosmetics according to any one of claims 1 to 10, wherein said surfactant is selected from anionic surfactants, cationic surfactants, ampholytic surfactants, nonionic surfactants, preferably wherein said surfactant is selected from the group of potassium myristate in aqueous solution, potassium laurate in aqueous solution, potassium palmitate in aqueous solution, potassium stearate in aqueous solution and/or polyethylene glycol monostearate.

12. Aerosol cosmetics comprising the composition for aerosol cosmetics according to any one of claims 1 to 11 and a propellant in an aerosol container, particularly wherein the propellant is a liquefied petroleum gas, carbon dioxide gas, nitrogen gas or nitrous oxide gas.

13. A method of producing a composition for aerosol cosmetics, comprising the step of mixing a solution comprising an organic solvent and water with hydroxypropyl methylcellulose to obtain the composition for aerosol cosmetics, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution.

14. A method of producing aerosol cosmetics, comprising the steps of mixing a solution comprising an organic solvent and water with hydroxypropyl methylcellulose to obtain the composition for aerosol cosmetics; and filling the composition for aerosol cosmetics and a propellant in an aerosol container to obtain the aerosol cosmetics, wherein a ratio of interfacial elastic modulus (E') to interfacial viscous modulus (E") of the hydroxypropyl methylcellulose is equal to or higher than 5.0 when measured with a tensiometer at 25°C using a 0.2% by mass aqueous solution, particularly wherein the internal pressure in the aerosol container is 0.4 MPa to 1.0 MPa at 25°C.

Europäisches
Patentamt

European
Patent Office

Office européen
des brevets

# EUROPEAN SEARCH REPORT

**Application Number**

EP 22 15 5125

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 632 219 A1 (BEIERSDORF AG [DE]) 8 March 2006 (2006-03-08) * paragraph [0001]; claim 1; example 3 * | 1-14 | INV. A61K8/04 A61K8/73 A61K8/34 |
| X | EP 1 340 485 A2 (OREAL [FR]) 3 September 2003 (2003-09-03) | 1-14 | A61K8/36 A61Q5/00 |
| Y | * claims 1,5,18,19; example 1 * | 1-14 | |
| X,D | JP 2019 059720 A (MANDOM CORP) 18 April 2019 (2019-04-18) * paragraph [0082]; claims 1,7 * | 1-14 | |
| X,P | EP 3 772 286 A1 (SHINETSU CHEMICAL CO [JP]) 10 February 2021 (2021-02-10) * claim 1; examples 3,6; tables 1-3 * | 1-9, 12-14 | |
| Y | EP 3 747 910 A1 (SHINETSU CHEMICAL CO [JP]) 9 December 2020 (2020-12-09) * claims; examples * | 1-14 | |

|  |
|---|
| **TECHNICAL FIELDS SEARCHED (IPC)** |
| A61K A61Q |

The present search report has been drawn up for all claims

| Place of search | Date of completion of the search | Examiner |
|---|---|---|
| Munich | 29 June 2022 | Verrucci, Marinella |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 15 5125

This annex lists the patent family members relating to the patent documents cited in the above-mentioned European search report.
The members are as contained in the European Patent Office EDP file on
The European Patent Office is in no way liable for these particulars which are merely given for the purpose of information.

29-06-2022

| Patent document cited in search report | | Publication date | Patent family member(s) | | Publication date |
|---|---|---|---|---|---|
| EP 1632219 | A1 | 08-03-2006 | CH | 697832 B1 | 27-02-2009 |
| | | | DE 102004041295 A1 | | 09-03-2006 |
| | | | EP | 1632219 A1 | 08-03-2006 |
| EP 1340485 | A2 | 03-09-2003 | AT | 385764 T | 15-03-2008 |
| | | | DE | 60319022 T2 | 05-02-2009 |
| | | | EP | 1340485 A2 | 03-09-2003 |
| | | | FR | 2836631 A1 | 05-09-2003 |
| | | | JP | 2004002319 A | 08-01-2004 |
| JP 2019059720 | A | 18-04-2019 | NONE | | |
| EP 3772286 | A1 | 10-02-2021 | CN | 112335735 A | 09-02-2021 |
| | | | EP | 3772286 A1 | 10-02-2021 |
| | | | JP | 2021027820 A | 25-02-2021 |
| | | | US | 2021037877 A1 | 11-02-2021 |
| EP 3747910 | A1 | 09-12-2020 | CN | 112048934 A | 08-12-2020 |
| | | | EP | 3747910 A1 | 09-12-2020 |
| | | | JP | 2020200457 A | 17-12-2020 |
| | | | KR | 20200140196 A | 15-12-2020 |
| | | | TW | 202120561 A | 01-06-2021 |
| | | | US | 2020385491 A1 | 10-12-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019059720 A

**Non-patent literature cited in the description**

- Viscosity Determination in General Tests. Japanese Pharmacopoeia
- Japanese Pharmacopoeia
- Loss on Drying Test. Japanese Pharmacopoeia
- **VISCOSITY DETERMINATION IN GENERAL TESTS.** Japanese Pharmacopoeia
- Hypromellose. Japanese Pharmacopoeia
- Loss on Drying Test in General Tests. Japanese Pharmacopoeia